# EUROPEAN PATENT SPECIFICATION

(11) **EP 1 598 018 B1**
(45) Date of publication and mention of the grant of the patent: **27.06.2012**
(21) Application number: 05010980.0
(22) Date of filing: 20.05.2005
(51) Int. Cl.: A61B 17/04, A61B 17/12

(54) **Treatment system for living tissues**
Behandlungssystem für lebendes Gewebe
Système de traitment pour tissus vivants

(30) Priority: 20.05.2004 US 572967 P
(43) Date of publication of application: 23.11.2005
(73) Proprietor: Olympus Corporation, Shibuya-ku, Tokyo (JP)
(72) Inventor: Sakamoto, Yuji c/o Olympus Corporation, Hachioji-shi Tokyo (JP)
(74) Representative: von Hellfeld, Axel

(56) References cited:
- WO-A-97/20522
- US-A- 4 235 238
- US-A1- 2003 236 535

## Description

The present invention relates to a treatment system for living tissues, which is used to perform endoscopic treatment, such as suturing or ligating an internal living tissue, in combination with an endoscope and other instruments.

A device that sutures or ligates an internal tissue under endoscopic observation, that is, a ligation treatment device used for endoscopic treatment, is disclosed in U.S. Pat. Appln. Publication Specification No. 2003/0236535A1 (hereinafter referred to as publication). The device disclosed in this publication can suture or ligate the tissue by penetrating a ligature having a fixing member through the tissue. In order to release a sutured or ligated state of the tissue, the ligature is cut or removed from the tissue with a fixing member grasped. If a desired region fails to be sutured or ligated, for example, the ligature is cut to release the sutured or ligated state. For example, a scissor forceps or the like is endoscopically used to cut the ligature. Besides, the fixing member is grasped and removed from the tissue by endoscopically using a grasping forceps or the like.

With the method disclosed in the above-identified reference, it is very complicated to operate the forceps and difficult to cut the ligature without damaging the living tissue when the ligature is buried in the tissue.

There is another method in which grasping forceps are used endoscopically to grasp tissue and then the stopper is removed. However, with this method, it is very complicated to operate the forceps when the stopper has such a shape that is difficult to grasp.

US 4,235,238 A discloses a suturing device having a suturing-finishing stop and a first stop, which are connected via a suturing thread.

The present invention has been proposed to solve the above-described drawbacks of the conventional techniques, and an object thereof is to provide a medical treatment device capable of suturing or ligating living tissue under endoscopic observation, and capable of releasing suruting or ligation of living tissue by a simple operation, as well as a treatment system for living tissue.

The invention is defined in claim 1.

According to an aspect of this invention, there is provided a medical treatment device includes a suturing-ligating member, a stopper, a fixing member and a suturing-ligation releasing member. The suturing-ligating member has a distal end portion, that carries out one of suturing and ligation of biological tissue by puncturing. The stopper is provided to be movable forward or backward on the suturing-ligating member, and stoppable by friction on the suturing-ligating member to maintain the biological tissue in a sutured or ligated state by the suturing-ligating member. The fixing member is provided on the distal end side of the suturing-ligating member to fix the distal end side of the suturing-ligating member to the biological tissue. The releasing member is provided on the distal end side of the suturing-ligating member to release the ligation state maintained by frictional stopping between the suturing-ligating member and the stopper by moving the suturing-ligating member to the distal end portion side with respect to the stopper.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a schematic sectional view of a treatment system for living tissues according to a first embodiment of this invention, showing a state in which a ligation treatment device, ligation instrument, and puncture needle are combined;
FIG. 2 is a schematic perspective view showing the ligation treatment device of the treatment system according to the first embodiment;
FIG. 3 is a schematic sectional view showing a state in which the puncture needle of the treatment system for living tissues according to the first embodiment is located close to a target tissue;
FIG. 4 is a schematic sectional view showing a state in which the puncture needle of the treatment system for living tissues according to the first embodiment is inserted through the target tissue;
FIG. 5 is a schematic sectional view showing a state in which a cylindrical member and a flexible wire of the ligation treatment device is disengaged from the puncture needle of the treatment system for living tissues according to the first embodiment;
FIG. 6 is a schematic sectional view showing the way the target tissue is ligated by means of the ligation treatment device of the treatment for living tissues according to the first embodiment;
FIG. 7 is a schematic perspective view showing a state in which a ligature on the proximal end side of a silicone tube is cut after the target tissue is ligated by means of the ligation treatment device of the treatment system for living tissues according to the first embodiment;
FIG. 8 is a schematic perspective view showing a state in which the flexible wire is grasped with a grasping forceps to release the ligation after the target tissue is ligated with the ligation treatment device of the treatment system for living tissues according to the first embodiment;
FIG. 9 is a schematic perspective view showing a state in which the flexible wire is grasped with the grasping forceps to release the ligation and the ligature is drawn out of the target tissue after the target tissue is ligated with the ligation treatment device of the treatment system for living tissues according to the first embodiment;
FIG. 10 is a schematic perspective view showing a state in which two separate tissues are sutured by means of the ligation treatment system for living tissues according to the first embodiment;
FIG. 11 is a schematic perspective view showing a ligation treatment device of a treatment system according to a second embodiment;
FIG. 12 is a schematic perspective view showing a ligation treatment device of a treatment system according to a third embodiment;
FIG. 13 is a schematic perspective view showing a ligation treatment device of a treatment system according to a fourth embodiment;
FIG. 14 is a schematic perspective view showing a ligation treatment device of a treatment system according to a fifth embodiment;
FIG. 15 is a schematic perspective view showing a ligation treatment device of a treatment system according to a sixth embodiment; and
FIG. 16 is a schematic perspective view showing a ligation treatment device of a treatment system according to a seventh embodiment.

Preferred embodiments of this invention will now be described with reference to the drawings.

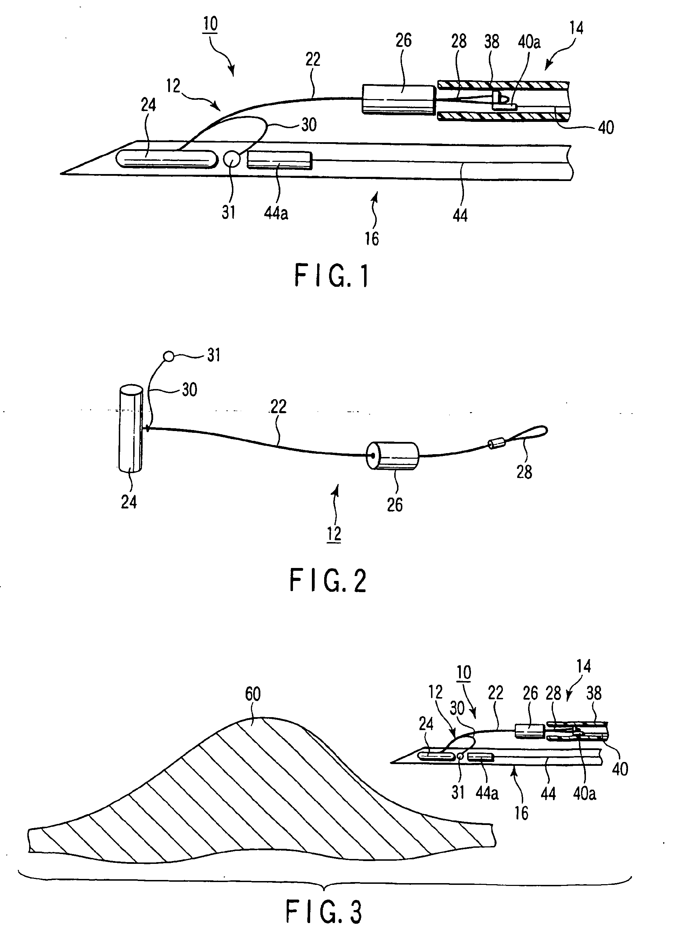

A first embodiment will first be described with reference to FIGS. 1 to 9.

A treatment system 10 for living tissues shown in FIG. 1 is a device for ligating living tissues, and is used for valve formation such that a tissue is ligated and bossed endoscopically, that is, by utilizing an endoscope, for example. This system 10 includes a ligation treatment device 12 for ligation treatment. It also includes a ligation instrument 14 and a puncture needle 16, which are used individually in combination with the ligation treatment device 12.

As shown in FIG. 2, the treatment device 12 includes a ligature (ligating member) 22, cylindrical member (ligature fixing member) 24, silicone tube (stopper) 26, proximal-end loop portion 28 formed on the proximal end portion of the ligature 22, and flexible wire 30.

The cylindrical member 24 is provided on the distal end portion of the ligature 22 that is used to ligate the tissue. The cylindrical member 24 bears one end portion that retains a desired position when the tissue is ligated with the ligature 22.

The ligature 22 is passed through the silicone tube 26. The silicone tube 26 is kept in a desired position relative to the ligature 22 by frictional force. It moves on the ligature 22 only when it is subjected to a given or greater force along the ligature 22. Thus, the silicone tube 26 serves as a stopper that keeps the ligature 22 in a desired position relative to the tissue when the tissue is ligated with the ligature 22. The silicone tube 26 bears the other end portion that, along with the cylindrical member 24, retains the desired position when the tissue is ligated with the ligature 22.

The flexible wire 30 is connected to the ligature 22 at a region near the distal end portion of the ligature 22. Although the flexible wire 30 is described as being one in number in connection with this embodiment, a plurality of flexible wires, e.g., two or three, may alternatively be connected to the ligature 22. If too many flexible wires 30 are connected to the ligature 22, they may possibly exert a bad influence, such as twining around the treatment device 12 when it is indwelt in a body cavity. Preferably, therefore, they should be one or two in number. Although the flexible wire 30 is described as being a wire rod in connection with this embodiment, it may alternatively be in the form of a belt.

The flexible wire 30 may be formed of any material that is flexible and strong enough to stand a pull when ligation is released. For example, it may be made of a stretched polyamide-based synthetic fiber, a material for a conventional suture, such as polypropylene, polyethylene terephthalate, or polytetrafluoroethylene, or a bioabsorbable material, such as polyglycol acid. This wire may be either a monofilament or a twisted yarn. The flexible wire 30 has a color that, unlike white, red, and yellow, does not resemble the colors of living tissues and is different from that of the ligature 22. Thereupon, an operator can favorably enjoy good visibility when he/she observes the flexible wire 30 through the endoscope.

As shown in FIG. 1, the ligation instrument 14 is provided with a ligation instrument sheath 38, a hook wire 40, and an operating handle (not shown). The hook wire 40 has a hook 40a on its distal end, which engages the proximal-end loop portion 28 of the ligature 22, and is passed through the ligation instrument sheath 38. The operating handle is provided at the proximal end portions of the ligation instrument sheath 38 and the hook wire 40, in order to manipulate the hook wire 40.

The puncture needle 16 is hollowed so that the cylindrical member 24 and the flexible wire 30 including an expanded portion 31 of the treatment device 12 can be stored in it. The puncture needle 16 has a pusher wire 44 in its bore, in order to push out the cylindrical member 24 and the flexible wire 30 from the distal end portion of the puncture needle 16 when the distal end portion of the puncture needle 16 is in a desired position. The distal end portion of the pusher wire 44 has a pusher 44a that can advance and retreat along the axis of the puncture needle 16. The puncture needle 16 is formed having a slit (not shown) through which the ligature 22 can be put into or taken out of the puncture needle 16.

The following is a description of operation according to the present embodiment. The operation described below is carried out endoscopically even in cases where no endoscope is illustrated. Described here are cases of ligating the target tissue 60 and releasing the ligation. The case of ligating the target tissue 60 will be described first.

As shown in FIG. 1, the ligation instrument 14 and the puncture needle 16 are combined with the treatment device 12 (see FIG. 2). The proximal-end loop portion 28 of the treatment device 12 is hitched to the hook 40a of the hook wire 40 of the ligation instrument 14. The silicone tube 26 of the treatment device 12 is caused to engage the distal end portion of the ligation instrument sheath 38 of the ligation instrument 14. The cylindrical member 24 and the flexible wire 30 including the expanded portion 31 of the treatment device 12 are loaded into the puncture needle 16.

As shown in FIG. 3, the puncture needle 16 of the treatment system 10 for living tissues in this state is brought close to the target tissue 60.

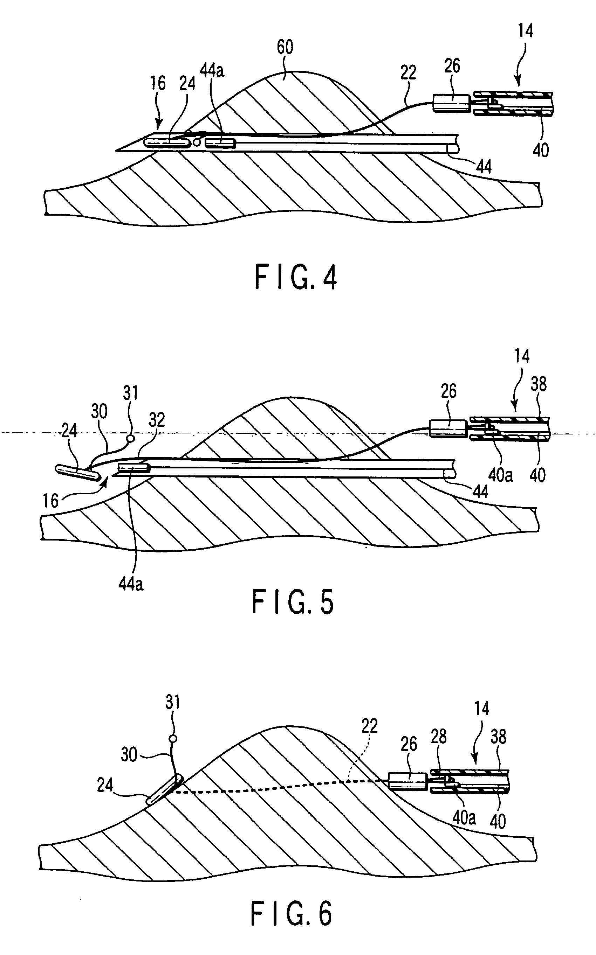

As shown in FIG. 4, the puncture needle 16 is inserted into target tissue 60, and the distal end portion of the puncture needle 16 is projected from the target tissue 60.

As shown in FIG. 5, the pusher wire 44 of the puncture needle 16 is advanced toward the distal end portion of the puncture needle 16, whereby the cylindrical member 24 and the flexible wire 30 are discharged to the outside of the puncture needle 16.

As shown in FIG. 6, the puncture needle 16 is drawn out of the tissue 60, and the ligature 22 is indwelt in the tissue 60. Since the cylindrical member 24 and the flexible wire 30 are located on the distal end portion of the ligature 22, the cylindrical member 24 and the flexible wire 30 are anchored to the tissue 60. If the hook wire 40 of the ligation instrument 14 is pulled with respect to the ligation instrument sheath 38, silicone tube 26 is pushed by the distal end portion of the ligation instrument sheath 38 as the silicone tube 26 is moved along the ligature 22 toward the distal end portion (or toward the cylindrical member 24). The target tissue 60 is held between the cylindrical member 24 and the silicone tube 26 as the tissue 60 is ligated. Movement of the silicone tube 26 relative to the ligature 22 is prevented by frictional force.

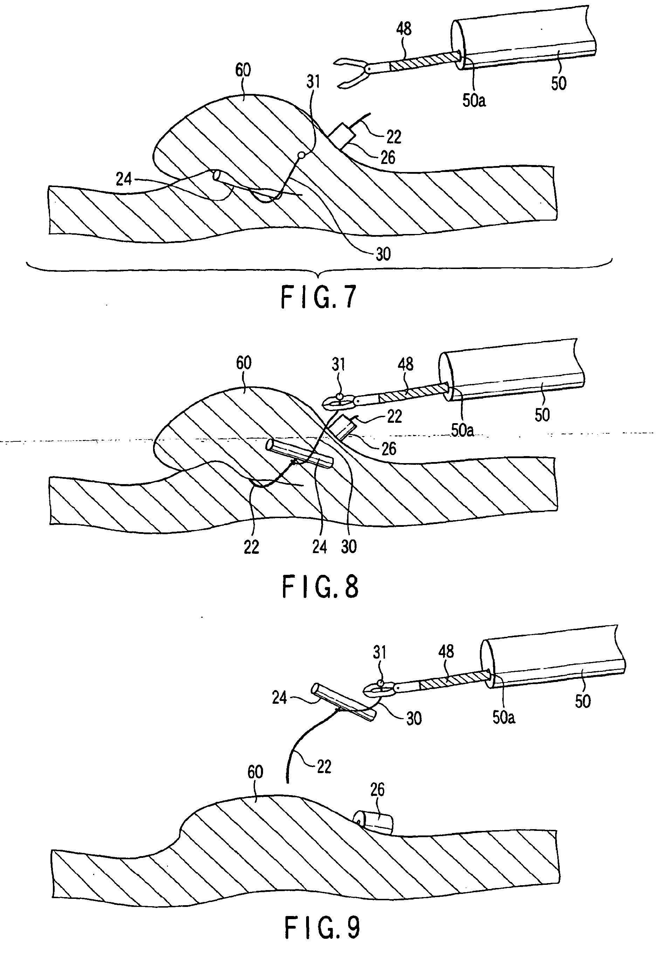

After the tissue 60 is ligated, as shown in FIG. 7, an odd of the ligature 22 on the proximal end side of the silicone tube 26 is cut.

The following is a description of the case of releasing the target tissue 60 from the ligation.

As shown in FIG. 8, an endoscope 50 used has a channel 50a through which a grasping forceps 48 can be passed. The flexible wire 30 is grasped with the grasping forceps 48 that is passed through the channel 50a, and the flexible wire 30 is pulled with a force that is greater than the frictional fixing force of the silicone tube 26. Since the flexible wire 30 is connected to the ligature 22 at the region near its distal end portion, the ligature 22 is trailed toward the distal end so that the cylindrical member 24 is moved away from the tissue 60. The ligature 22 that is connected to the cylindrical member 24 is also drawn out of the tissue 60 and disengaged from the tissue 60.

As shown in FIG. 9, a sutured or ligated state is released, whereupon the silicone tube 26 comes off the ligature 22. The cylindrical member 24 must have a size such that it can be stored in the puncture needle 16. If the tissue 60 is released from the ligation by causing the silicone tube 26 to come off the proximal end side of the ligature 22, therefore, the cylindrical member 24 may possibly be buried in the tissue 60. If the silicone tube 26 is made large enough not to be buried in the tissue 60, according to this embodiment, the ligation of the tissue 60 is released from the distal end portion side (side of the cylindrical member 24) of the ligature 22. Thus, the cylindrical member 24 can be prevented from being buried in the tissue 60 when the ligation is released.

According to this embodiment, as described above, the following effects can be enjoyed.

In releasing the tissue 60 from the ligation, the flexible wire 30 is grasped with the grasping forceps 48. Therefore, the flexible wire 30 can be grasped more easily through the endoscope 50 than when the cylindrical member 24 is grasped directly. Thus, the tissue 60 can be prevented from being wrongly influenced. Since the expanded portion 31 is provided on the distal end of the flexible wire 30, the flexible wire 30 can be prevented from failing to be seized when it is grasped with the grasping forceps 48.

The ligature 22 can be drawn out from the silicone tube 26, as well as from the tissue 60, without regard to the direction in which the flexible wire 30 is pulled when the flexible wire 30 is grasped with the grasping forceps 48.

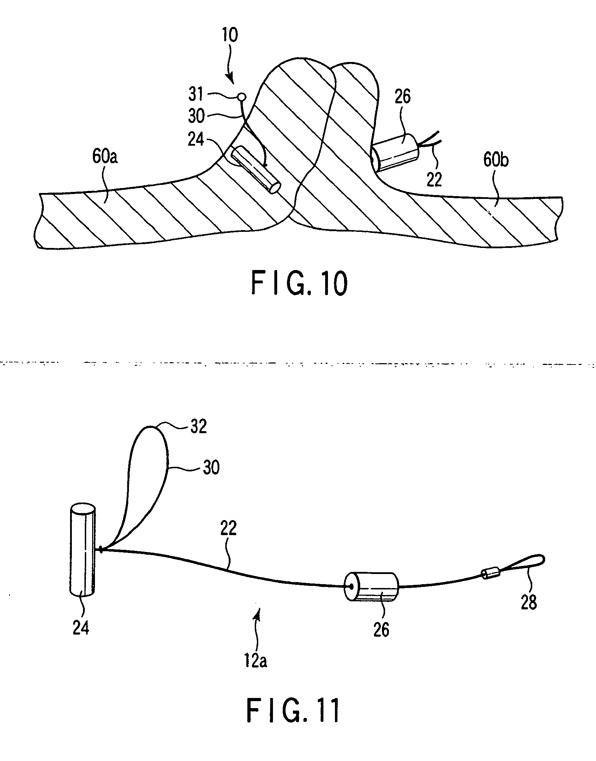

As shown in FIG. 10, the treatment system 10 for living tissues according to this embodiment may be also used to suture, for example, two tissues 60a and 60b that are separate from each other. For example, it can suture and close perforations such as ulcers in the tissues 60a and 60b.

A second embodiment will now be described with reference to FIG. 11. This embodiment is a modification of the first embodiment. Like numerals are used to designate like members described in connection with the first embodiment, and a detailed description of those members is omitted.

As shown in FIG. 11, a ligation treatment device 12a according to this embodiment differs from the ligation treatment device 12 (see FIG. 2) according to the first embodiment in that a flexible wire 30 is connected to a ligature 22 by means of a loop-shaped loop portion 32.

The same functions and effects of the first embodiment can be obtained with use of this treatment device 12a. Besides, the flexible wire 30 has the loop portion 32, so that it can be also held by being hooked by the grasping forceps 48, for example. Thus, the flexible wire 30 can be kept easily graspable. Since the loop portion 32 doubles the flexible wire 30 that is grasped by means of the grasping forceps 48, pull force that is obtained with use of the grasping forceps 48 can be made greater than in the case where the flexible wire 30 is single (see FIG. 2).

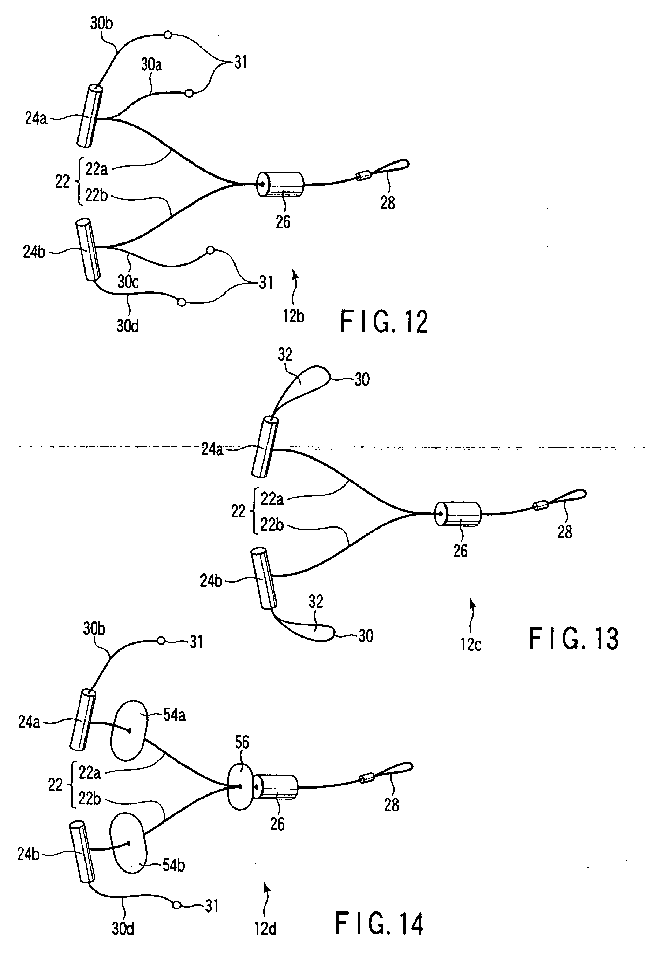

A third embodiment will now be described with reference to FIG. 12. This embodiment is a modification of the first embodiment. Like numerals are used to designate like members described in connection with the first embodiment, and a detailed description of those members is omitted.

As shown in FIG. 12, a ligation treatment device 12b according to this embodiment differs from the ligation treatment device 12 (see FIG. 2) according to the first embodiment in a plurality of particulars.

Two ligatures 22 are passed through a silicone tube 26. More specifically, the ligatures 22 include a first ligature 22a and a second ligature 22b. The first and second ligatures 22a and 22b are connected to each other at their respective proximal end portions, and a proximal-end loop portion 28 is formed on the proximal end portions of the ligatures 22a and 22b. A first cylindrical member 24a is provided on the distal end portion of the first ligature 22a. A second cylindrical member 24b is provided on the distal end portion of the second ligature 22b.

Flexible wires 30a and 30b having an expanded portion 31 each are connected to the central portion (ligature junction) and a side end portion, respectively, of the first cylindrical member 24a. Thus, the flexible wires 30a and 30b are connected to the first cylindrical member 24a, not to the ligature 22. Flexible wires 30c and 30d having an expanded portion 31 each are connected to the central portion (ligature junction) and a side end portion, respectively, of the second cylindrical member 24b. Thus, the flexible wires 30c and 30d are connected to the second cylindrical member 24b, not to the ligature 22.

The first and second cylindrical members 24a and 24b and the flexible wires 30a, 30b, 30c and 30c having their respective expanded portions 31 can be stored in the puncture needle 16.

The same functions and effects of the first embodiment can be obtained with use of this treatment device 12b. Besides, the flexible wires 30a, 30b, 30c and 30d extend from positions remoter from the tissue 60 when the tissue 60 is ligated, so that the flexible wires 30a, 30b, 30c and 30d can difficulty stick to the tissue 60, and the flexible wires 30a, 30b, 30c and 30d can be easily grasped with the grasping forceps 48. Since the flexible wires 30a, 30b, 30c and 30d are connected to the first and second cylindrical members 24a and 24b that are exposed on the surface of the tissue 60, the flexible wires 30a, 30b, 30c and 30d can be difficultly buried in the tissue 60.

A fourth embodiment will now be described with reference to FIG. 13. This embodiment is a modification of the third embodiment. Like numerals are used to designate like members described in connection with the third embodiment, and a detailed description of those members is omitted.

As shown in FIG. 13, a ligation treatment device 12c according to this embodiment differs from the ligation treatment device 12b (see FIG. 12) according to the third embodiment in that flexible wires 30 have their respective loop-shaped loop portions 32 and are connected individually to respective the side end portions of first and second cylindrical members 24a and 24b.

The same functions and effects of the third embodiment can be obtained with use of this treatment device 12c. Besides, the flexible wires 30 have the loop portions 32, so that they can be also held by being hooked by the grasping forceps 48, for example. Thus, the flexible wires 30 can be kept easily graspable. Since the loop portions 32 double the flexible wires 30 that are grasped by means of the grasping forceps 48, pull force that is obtained with use of the grasping forceps 48 can be made greater than in the case where the flexible wire 30 is single (see FIG. 2).

A fifth embodiment will now be described with reference to FIG. 14. This embodiment is a modification of the third embodiment. Like numerals are used to designate like members described in connection with the third embodiment, and a detailed description of those members is omitted.

As shown in FIG. 14, a ligation treatment device 12d according to this embodiment differs from the ligation treatment device 12b (see FIG. 12) according to the third embodiment in a plurality of particulars.

A first distal-side pledget 54a is located between a first cylindrical member 24a and a silicone tube 26 and near the first cylindrical member 24a in a manner such that it is penetrated by a first ligature 22a. The first distal-side pledget 54a is formed with a hole in its central portion, through which the first ligature 22a is passed. Likewise, a second distal-side pledget 54b is located between a second cylindrical member 24b and the silicone tube 26 and near the second cylindrical member 24b in a manner such that it is penetrated by a second ligature 22b. The second distal-side pledget 54b is formed with a hole in its central portion, through which the second ligature 22b is passed.

A proximal-side pledget 56 is located between the silicone tube 26 and the first and second distal-side pledgets 54a and 54b and near the silicone tube 26 in a manner such that it is penetrated by the first and second ligatures 22a and 22b. The proximal-side pledget 56 is formed with a hole in its central portion, through which the first and second ligatures 22a and 22b are passed. The first and second distal-side pledgets 54a and 54b and the proximal-side pledget 56 can be stored in a puncture needle 34. Thus, the tissue 60 is held between the proximal-side pledget 56 and the first and second distal-side pledgets 54a and 54b when it is ligated.

A flexible wire 30b is connected to a side end portion of the first cylindrical member 24a. A flexible wire 30d is connected to a side end portion of the second cylindrical member 24b. Flexible wires 30a and 30c (see FIG. 12) are not connected to the respective central portions (ligature junctions) of the first and second cylindrical members 24a and 24b.

The same functions and effects of the third embodiment can be obtained with use of this treatment device 12d. Besides, the first and second distal-side pledgets 54a and 54b increase the area of contact with the tissue 60 when the tissue 60 is ligated, so that the first and second cylindrical members 24a and 24b can be securely prevented from being buried in the tissue 60. Since the proximal-side pledget 56 increases the area of contact with the tissue 60 when the tissue 60 is ligated, the silicone tube 26 can be securely prevented from being buried in the tissue 60.

Since the first and second cylindrical members 24a and 24b are exposed on the tissue 60 without being buried in the tissue 60, the flexible wires 30 are not buried in the tissue 60, so that the flexible wires 30b and 30d can be kept easily graspable by means of the grasping forceps 48.

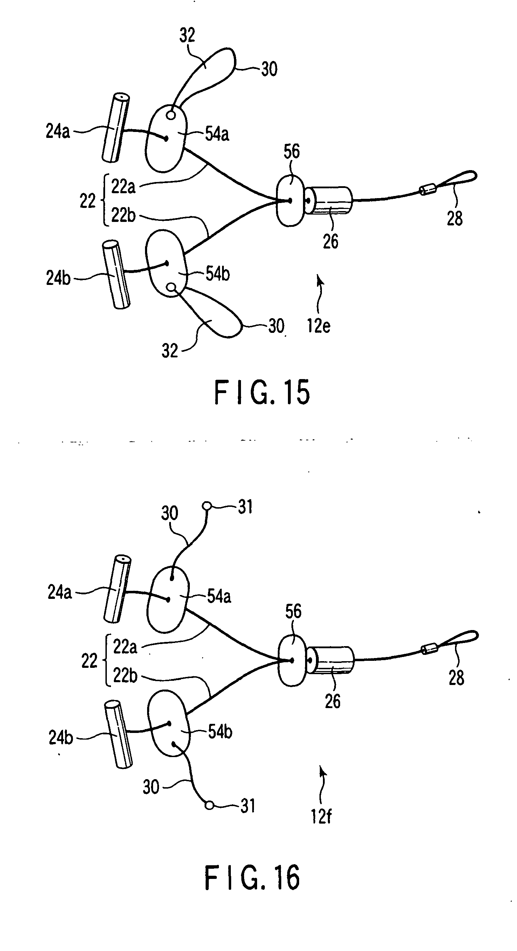

A sixth embodiment will now be described with reference to FIG. 15. This embodiment is a modification of the fifth embodiment. Like numerals are used to designate like members described in connection with the fifth embodiment, and a detailed description of those members is omitted.

As shown in FIG. 15, a ligation treatment device 12e according to this embodiment differs from the ligation treatment device 12d (see FIG. 14) according to the fifth embodiment in a plurality of particulars.

Flexible wires 30 are attached to first and second distal-side pledgets 54a and 54b, individually. The flexible wires 30 have their respective loop-shaped loop portions 32, which are passed through holes in the respective end portions of the first and second distal-side pledgets 54a and 54b, individually.

The same functions and effects of the fifth embodiment can be obtained with use of this treatment device 12e. Besides, the flexible wires 30 are connected to the respective end portions of the first and second distal-side pledgets 54a and 54b, so that they can be difficulty subjected to pressure between the tissue 60 and first and second cylindrical members 24a and 24b. Thus, the first and second cylindrical members 24a and 24b can be difficulty buried in the tissue 60, so that the flexible wire 30 can be kept easily graspable.

Since the flexible wires 30 have the loop portions 32, they can be also held by being hooked by the grasping forceps 48, for example, so that the flexible wires 30 can be kept easily graspable. Since the loop portions 32 double the flexible wires 30 that are grasped by means of the grasping forceps 48, pull force that is obtained with use of the grasping forceps 48 can be made greater than in the case where the flexible wire 30 is single (see FIG. 14).

A seventh embodiment will now be described with reference to FIG. 16. This embodiment is a modification of the sixth embodiment. Like numerals are used to designate like members described in connection with the sixth embodiment, and a detailed description of those members is omitted.

As shown in FIG. 16, a ligation treatment device 12f according to this embodiment differs from the ligation treatment device 12e (see FIG. 15) according to the sixth embodiment in that flexible wires 30 having expanded portions 31 on their respective distal ends are connected to first and second distal-side pledgets 54a and 54b, individually.

With use of this treatment device 12f, compared with the sixth embodiment, the flexible wires 30 are shaped so that they are not space-consuming when the treatment device 12f is endoscopically indwelt in a human body. Thus, food or the like being deglutition or the endoscope 50 or the grasping forceps 48 being disengaged can be prevented from catching the flexible wires 30.

## Claims

1. A medical treatment device (12) comprising:
a suturing-ligating member (22; 22a, 22b) having a distal end portion and a proximal end portion, that carries out one of suturing and ligation of biological tissue (60; 60a, 60b) by puncturing;
a stopper (26) provided to be movable forward or backward on the suturing-ligating member, and stoppable by friction on the suturing-ligating member to maintain the biological tissue in a sutured or ligated state by the suturing-ligating member; and
a fixing member (24; 24a, 24b) provided on the distal end side of the suturing-ligating member to fix the distal end side of the suturing-ligating member to the biological tissue,
**characterized by** a suturing-ligation releasing member (30; 30a, 30b, 30c, 30d) provided on the distal end side of the suturing-ligating member to release the ligation state maintained by frictional stopping between the suturing-ligating member and the stopper by moving the suturing-ligating member to the distal end portion side with respect to the stopper, wherein the suturing-ligation releasing member (30; 30a, 30b, 30c, 30d) is made of a flexible wire material.

2. The medical treatment device (12) according to claim 1, **characterized in that** the suturing-ligation releasing member (30; 30a, 30b, 30c, 30d) is provided on the fixing member (24; 24a, 24b).

3. The medical treatment device (12) according to claim 1, **characterized in that** the suturing-ligation releasing member (30; 30a, 30b, 30c, 30d) comprises:
a first end portion fixedly mounted to a pledget (54; 54a, 54b) provided on the distal end side, which is opposite to the fixing member (24; 24a, 24b) to prevent the stopper (26) from digging into the biological tissue; and a second end portion located on an opposite side to the first end portion and equipped with a to-be-grasped portion.

4. The treatment device (12) according to claim 3, **characterized in that** the suturing-ligation releasing member (30; 30a, 30b, 30c, 30d) has an expanded part (31) in at least one portion thereof.

5. The treatment device (12) according to claim 1 to 4, **characterized in that** the suturing-ligation releasing member (30; 30a, 30b, 30c, 30d) has a loop (32).

6. A treatment system for living tissue **characterized by** comprising a medical treatment device (12) according any one of claims 1 to 5, the system further comprising:
a treatment Instrument (14) to be used in combination with the medical treatment device; and
a hollow puncturing needle (16) to be used in combination with the medical treatment device,
the treatment instrument further comprising:
a tube-shaped sheath (38) having a distal end configured to abut to the stopper (26);
a hook wire (40) pierced through the sheath and having a hook (40a) configured to hook to the proximal end of the suturing-ligation member (22; 22a, 22b); and
an operation handle that moves the hook wire forward and backward with respect to the sheath (38);
wherein the puncturing needle is configured to house the fixing member (24; 24a, 24b) and the suturing-ligation releasing member (30; 30a, 30b, 30c, 30d) and contains a pusher (44a) that pushes the fixing member and the suturing-ligation releasing member with respect to the puncturing needle within a hollow space thereinside.

## Patentansprüche

1. Medizinische Behandlungsvorrichtung (12) mit:
einem Näh-Abbindeelement (22; 22a, 22b) mit einem distalen Endabschnitt und einem proximalen Endabschnitt, das entweder einen Näh- oder einen Abbindevorgang von biologischem Gewebe (60; 60a, 60b) durch Punktieren ausführt;
einem Feststellteil (26), das vorwärts oder rückwärts beweglich an dem Näh-Abbindeelement vorgesehen ist, und das durch Reibung an dem Näh-Abbindeelement feststellbar ist, um das biologische Gewebe in einem durch das Näh-Abbindeelement genähten oder abgebundenen Zustand zu halten; und
einem Fixierelement (24; 24a, 24b), das an der distalen Endseite des Näh-Abbindeelements vorgesehen ist, um die distale Endseite des Näh-Abbindeelements an dem biologischen Gewebe zu fixieren,
**gekennzeichnet durch** ein Näh-Abbinde-Freigabeelement (30; 30a, 30b, 30c, 30d), das an der distalen Endseite des Näh-Abbindeelements vorgesehen ist, um den durch Reibschluss zwischen dem Näh-Abbindeelement und dem Feststellteil aufrechterhaltenen Abbindezustand **durch** Bewegen des Näh-Abbindeelements zu dem distalen Endabschnitt in Bezug auf das Feststellteil freizugeben, wobei das Näh-Abbinde-Freigabeelement (30; 30a, 30b, 30c, 30d) aus einem biegsamen Drahtmaterial gebildet ist.

2. Medizinische Behandlungsvorrichtung (12) nach Anspruch 1, **dadurch gekennzeichnet, dass** das Näh-Abbinde-Freigabeelement (30; 30a, 30b, 30c, 30d) an dem Fixierelement (24; 24a, 24b) vorgesehen ist.

3. Medizinische Behandlungsvorrichtung (12) nach Anspruch 1, **dadurch gekennzeichnet, dass** das Näh-Abbinde-Freigabeelement (30; 30a, 30b, 30c, 30d) umfasst:
einen ersten Endabschnitt, der fest an einem an der distalen Endseite vorgesehenen Pledget (54; 54a, 54b) angebracht ist, das dem Fixierelement (24; 24a, 24b) gegenüberliegt, um zu verhindern, dass sich das Feststellteil (26) in das biologische Gewebe eingräbt; und einen zweiten Endabschnitt, der sich an einer dem ersten Endabschnitt gegenüberliegenden Seite befindet und mit einem zu greifenden Abschnitt ausgestattet ist.

4. Behandlungsvorrichtung (12) nach Anspruch 3, **dadurch gekennzeichnet, dass** das Näh-Abbinde-Freigabeelement (30; 30a, 30b, 30c, 30d) einen Verdickungsteil (31) in mindestens einem Abschnitt desselben aufweist.

5. Behandlungsvorrichtung (12) nach Anspruch 1 bis 4, **dadurch gekennzeichnet, dass** das Näh-Abbinde-Freigabeelement (30; 30a, 30b, 30c, 30d) eine Schlinge (32) aufweist.

6. Behandlungssystem für lebendes Gewebe, **dadurch gekennzeichnet, dass** es eine medizinische Behandlungsvorrichtung (12) nach einem der Ansprüche 1 bis 5 aufweist, wobei das System ferner umfasst:
ein Behandlungsinstrument (14), das in Kombination mit der medizinischen Behandlungsvorrichtung anzuwenden ist; und
eine hohle Punktionsnadel (16), die in Kombination mit der medizinischen Behandlungsvorrichtung anzuwenden ist,
wobei das Behandlungsinstrument ferner umfasst:
eine schlauchförmige Hülse (38) mit einem distalen Ende, das konfiguriert ist, um an dem Feststellteil (26) anzuliegen;
einen Hakendraht (40), der die Hülse durchsetzt und einen Haken (40a) hat, der konfiguriert ist, um sich an dem proximalen Ende des Näh-Abbindeelements (22; 22a, 22b) einzuhaken; und
einen Betätigungsgriff, der den Hakendraht in Bezug auf die Hülse (38) vorwärts und rückwärts bewegt;
wobei die Punktionsnadel konfiguriert ist, um das Fixierelement (24; 24a, 24b) und das Näh-Abbinde-Freigabeelement (30; 30a, 30b, 30c, 30d) aufzunehmen, und einen Schieber (44a) enthält, der das Fixierelement und das Näh-Abbinde-Freigabeelement bezüglich der Punktionsnadel in einem Hohlraum in dieser verschiebt.

## Revendications

1. Dispositif de traitement médical (12), comprenant :
un élément de suture-ligature (22 ; 22a, 22b) comportant une partie d'extrémité distale et une partie d'extrémité proximale, qui exécute, par ponction, l'une d'une suture et d'une ligature d'un tissu biologique (60 ; 60a, 60b) ;
un élément d'arrêt (26) conçu pour être mobile vers l'avant ou vers l'arrière sur l'élément de suture-ligature, et pouvant être arrêté par friction sur l'élément de suture-ligature dans le but de maintenir le tissu biologique dans un état suturé ou ligaturé par l'élément de suture-ligature ; et
un élément de fixation (24 ; 24a, 24b) disposé du côté d'extrémité distale de l'élément de suture-ligature dans le but de fixer le côté d'extrémité distale de l'élément de suture-ligature par rapport au tissu biologique,
**caractérisé par** un élément de libération de suture-ligature (30 ; 30a, 30b, 30c, 30d) disposé du côté d'extrémité distale de l'élément de suture-ligature dans le but de libérer l'état ligaturé maintenu par l'arrêt par friction entre l'élément de suture-ligature et l'élément d'arrêt, en amenant l'élément de suture-ligature vers le côté de partie d'extrémité distale par rapport à l'élément d'arrêt, dans lequel l'élément de libération de suture-ligature (30 ; 30a, 30b, 30c, 30d) est réalisé en matériau de fil souple.

2. Dispositif de traitement médical (12) selon la revendication 1, **caractérisé en ce que** l'élément de libération de suture-ligature (30 ; 30a, 30b, 30c, 30d) est disposé sur l'élément de fixation (24 ; 24a, 24b).

3. Dispositif de traitement médical (12) selon la revendication 1, **caractérisé en ce que** l'élément de libération de suture-ligature (30 ; 30a, 30b, 30c, 30d) comprend :
une première partie d'extrémité montée de manière fixe sur un bourdonnet (54 ; 54a, 54b) disposé du côté d'extrémité distale, qui est opposé à l'élément de fixation (24 ; 24a, 24b) pour empêcher que l'élément d'arrêt (26) ne creuse le tissu biologique ; et une seconde partie d'extrémité située d'un côté opposé à la première partie d'extrémité et équipée d'une partie à saisir.

4. Dispositif de traitement (12) selon la revendication 3, **caractérisé en ce que** l'élément de libération de suture-ligature (30 ; 30a, 30b, 30c, 30d) comporte une partie plus volumineuse (31) dans au moins l'une de ses parties.

5. Dispositif de traitement (12) selon les revendications 1 à 4, **caractérisé en ce que** l'élément de libération de suture-ligature (30 ; 30a, 30b, 30c, 30d) comporte une boucle (32).

6. Système de traitement d'un tissu vivant, **caractérisé par le fait qu'**il comprend un dispositif de traitement médical (12) selon l'une quelconque des revendications 1 à 5, le système comprenant en outre :
un instrument de traitement (14) à utiliser en combinaison avec le dispositif de traitement médical ; et
une aiguille creuse de ponction (16) à utiliser en combinaison avec le dispositif de traitement médical,
l'instrument de traitement comprenant en outre :
une gaine en forme de tube (38) comportant une extrémité distale configurée pour venir en butée sur l'élément d'arrêt (26) ;
un fil d'accrochage (40) traversant la gaine et comportant un crochet (40a) configuré pour accrocher l'extrémité proximale de l'élément de suture-ligature (22 ; 22a, 22b) ; et
une poignée de manipulation qui déplace le fil d'accrochage vers l'avant et vers l'arrière par rapport à la gaine (38) ;
dans lequel l'aiguille de ponction est configurée pour loger l'élément de fixation (24 ; 24a, 24b) et l'élément de libération de suture-ligature (30 ; 30a, 30b, 30c, 30d) et contient un poussoir (44a) qui pousse l'élément de fixation et l'élément de libération de suture-ligature par rapport à l'aiguille de ponction à l'intérieur d'un espace creux réalisé dans celle-ci.
